# EUROPÄISCHE PATENTSCHRIFT

(11) **EP 2 238 908 B1**
(45) Veröffentlichungstag und Bekanntmachung des Hinweises auf die Patenterteilung: **24.08.2011**
(21) Anmeldenummer: 10158136.1
(22) Anmeldetag: 29.03.2010
(51) Int. Cl.: A61B 6/03, H01J 35/30

(54) **Anordnung zur Elektronenstrahltomographie**
Electron beam tomography arrangement
Arrangement de tomodensitomètre à faisceau électronique

(30) Priorität: 01.04.2009 DE 102009002114
(43) Veröffentlichungstag der Anmeldung: 13.10.2010
(73) Patentinhaber: Helmholtz-Zentrum Dresden - Rossendorf e.V., 01328 Dresden (DE)
(72) Erfinder: Hampel, Uwe, 01324, Dresden (DE)

(56) Entgegenhaltungen:
- DE-A1-102007 019 176
- US-A- 4 352 021
- US-A- 4 531 226
- US-B1- 7 023 950

## Beschreibung

Die Erfindung betrifft eine Anordnung zur Röntgen-Computertomographie mit einem gescannten Elektronenstrahl.

Elektronenstrahltomographie wird heute für Strömungsuntersuchungen von Mehrphasenströmungen und in der medizinischen Diagnostik, insbesondere zur Bildgebung des schlagenden Herzens eingesetzt (Fischer et al., Meas. Sci. Technol. 19, pp. 094002, 2008). Dabei wird ein in einer Vakuumkammer geführter Elektronenstrahl mittels eines elektromagnetischen Ablenksystems über ein teilkreisförmiges Metalltarget geführt, womit ein schnell beweglicher Röntgenbrennfleck erzeugt wird. Ein mit leichtem axialem Versatz zum Target angeordneter kreis- oder teilkreisförmiger Röntgendetektorbogen erfasst die das Untersuchungsobjekt durchdringende Röntgenstrahlung. Aus dessen Messdaten kann durch Anwendung tomographischer Bildrekonstruktionsverfahren die Materialverteilung in der durchstrahlten Schnittebene berechnet werden.

Der besondere Vorteil der Elektronenstrahltomographie liegt in der hohen erreichbaren Bildrate, die durch die schnelle Ablenkbarkeit des trägheitsfreien Elektronenstrahls mit Hilfe magnetischer Wechselfelder gegeben ist. Im Prinzip lassen sich mit dieser Anordnung zunächst nur Schnittbilder einer Durchstrahlungsebene erzeugen. Aufnahmen verschiedener Schichten des Untersuchungsobjektes sind prinzipiell durch axiale Bewegung von Untersuchungsobjekt oder Scanner möglich. Allerdings benötigt eine solche mechanische Bewegung zu viel Zeit, um schnelle Veränderungen des Untersuchungsobjektes in verschiedenen Durchstrahlungsebenen gleichzeitig abzubilden. Eine quasi gleichzeitige Aufnahme von Schnittbildern in verschiedenen Durchstrahlungsebenen ist beispielsweise mit dem in der Schrift US 4 352 021 A offengelegten Elektronenstrahl-Scanner möglich. Bei diesem kann der Elektronenstrahl nacheinander über verschiedene, in axialer Richtung hintereinander liegende, Targetsegmente geführt werden. Da bei dieser Anordnung aber nur ein einzelner Röntgendetektorbogen zur Anwendung kommt, muss der Schnittebenenabstand klein gehalten werden, um axiale Abbildungsunschärfen zu vermeiden, die aus der nicht koplanaren Anordnung von Brennfleckbahn und Röntgendetektorbogen resultieren. Ein weiterer Nachteil dieser Anordnung besteht darin, dass die Aufnahme von Bildern in unterschiedlichen axialen Schnittebenen nacheinander und nicht gleichzeitig erfolgt. Damit können während der Scanbewegung des Elektronenstrahls über dem einer Durchstrahlungsebene zugeordneten Targetbereich keine Informationen über die Materialverteilung in den übrigen Durchstrahlungsebenen aufgezeichnet werden. Besonders für Anwendungen der Elektronenstrahltomographie zur Untersuchungen schneller Prozesse, wie etwa Mehrphasenströmungen, ist dies ein entscheidender Nachteil, da das sequentielle Abscannen von Ebenen zu Informationsverlusten führt.

DE 10 2007 019 176 A1 beschreibt einen Computertomographen mit diskreten Fokusflächen auf dem Target, die sägezahnförmig angeordneten sind. Der Nachteil dieser Anordnung ist, dass die Fokusflächen aufwendig produziert werden müssen und die Anordnung sehr kompliziert erscheint, weil die einzelnen Flächen justiert werden müssen.

US 7,023,950 B1 beschreibt eine Anordnung zur Durchstrahlung von zu untersuchen Stoffen, Produkten oder Erzeugnissen mit Röntgenstrahlen, wobei das Target in Abschnitte (z. B. durch Aufbringen von hoch-emittierendem Material), so dass der Ort des Röntgenstrahls bestimmt werden kann.

Aufgabe der vorliegenden Erfindung ist es, eine Anordnung zur Elektronenstrahltomographie anzugeben, die einen kontinuierlichen Mehrebenen-Scan mit quasi zeitgleicher Aufnahme von Schnittbildern mehrerer Durchstrahlungsebenen eines Untersuchungsobjektes mit hoher zeitlicher und räumlicher Auflösung gestattet.

Zur Lösung der Aufgabe wird eine Anordnung zur Elektronenstrahltomographie mit einer Vielzahl speziell geformter und hintereinander angeordneter Targets sowie einer Vielzahl feststehender Röntgendetektorbögen angegeben. Die Aufgabe wird durch die Merkmale des Anspruchs 1 gelöst. Ausgestaltungen der Erfindung sind in den Unteransprüchen ausgeführt.

Der besondere Vorteil der erfundenen Anordnung zur Elektronenstrahltomographie besteht darin, dass mit ihr die gleichzeitige Aufnahme zweidimensionaler Schnittbilder eines Objektes in verschiedenen Durchstrahlungsebenen mit hoher Bildrate möglich ist. Damit ist die erfundene Anordnung für viele diagnostische Fragestellungen geeignet, bei denen dynamische Vorgänge untersucht werden sollen. Ein Beispiel dafür ist die Vermessung von Mehrphasenströmungen, wo mittels einer schnellen und zeitgleichen Bildgebung in zwei oder mehr Durchstrahlungsebenen die Geschwindigkeiten von Strukturen in der Strömung bestimmt werden können.

Die Erfindung wird nachfolgend anhand eines Ausführungsbeispiels näher erläutert. In den zugehörigen Zeichnungen zeigen
Fig. 1 Ansicht der erfindungsgemäßen Anordnung,
Fig. 2 Ansicht der erfindungsgemäßen Targetanordnung.

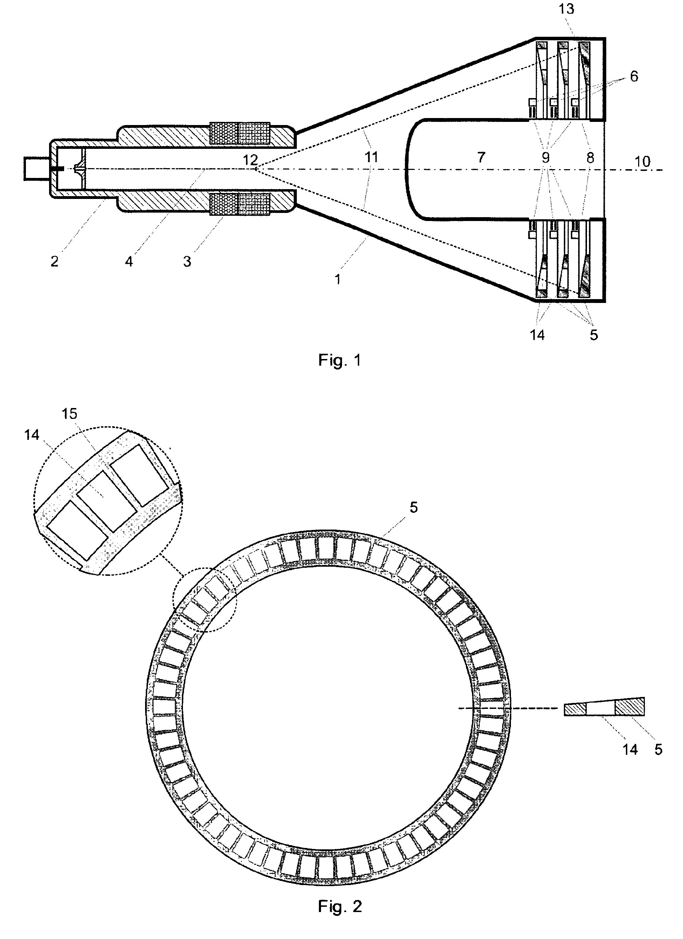

Die erfundene Anordnung (Fig. 1) besteht mindestens aus folgenden Komponenten:
- einer Vakuumkammer (**1**), die das Elektronenstrahlsystem umschließt,
- einer Elektronenkanone (**2**),
- einem elektronenoptischen System (**3**) zur Fokussierung und zweidimensionalen Ablenkung des aus der Elektronenkanone (**2**) austretenden Elektronenstrahls (**4**),
- mehreren speziell geformten Targets (**5**) zur Röntgenstrahlerzeugung,
- sowie mehreren Röntgendetektorbögen (**6**),
- einer Aufnahmeöffnung für das Untersuchungsobjekt (**7**),
- bei der im Innenbereich die Wandung der Vakuumkammer (**1**) zu einem Röntgenstrahlaustrittsfenster (**8**) ausgedünnt ist und
- Detektorblenden (**9**).
   Weitere optional hier nicht explizit aufgeführte, aber dem Fachmann bekannte Komponenten sind
- Sensoren zur Messung und Überwachung wichtiger Betriebsparameter (Targettemperatur, Vakuumgüte, Elektronenstrahlparameter),
- Elektronikkomponenten zur Messwertertassung, Hoch- und Hilfsspannungserzeugung und Strahlführung, Komponenten zur Strahlaufhärtung,
- Komponenten zur Abschirmung,
- Liege oder Halterungen für das Untersuchungsobjekt,
- Kühlvorrichtung für das Target, etc.

Im unabgelenkten Zustand verlässt der Elektronenstrahl (**4**) die Elektronenkanone (**2**) geradlinig durch die Anodenbohrung. Die gedachte Gerade von der Kathode (Elektronenquelle) zur Anodenbohrung wird forthin als optische Achse (**10**) bezeichnet. Der aus der Elektronenkanone (**2**) austretende Elektronenstrahl (**4**) wird durch das elektronenoptische System (**3**) aus der Richtung der optischen Achse (**10**) um einen Winkel α ausgelenkt und um die optische Achse (**10**) rotiert. Dadurch läuft der Elektronenstrahl (**4**) auf der gedachten Mantelfläche eines Elektronenstrahlkegels (**11**) dessen Spitze der Ablenkpunkt (**12**) des Elektronenstrahls (**4**) im Ablenkspulensystem des elektronenoptischen Systems (3) ist. Allgemein ist die zeitabhängige Auslenkung durch die mathematische Funktion α(ω*t*) mit der Kreisfrequenz ω der Rotation gegeben. Für ring- oder teilringförmige Targets (**5**), die senkrecht zur optischen Achse (**10**) stehen, wird der Elektronenstrahl (**4**) kreisförmig um die optische Achse (**10**) rotiert, so dass der auf einem Target (**5**) entstehende Brennfleck (**13**) sich auf einer Kreisbahn bewegt.

Im kopfseitigen Teil der Vakuumkammer (**1**) sind die teil- oder vollringförmigen Targets (5) in Elektronenstrahlrichtung hintereinander liegend angeordnet. Die Targets (**5**) sind Metallringe mit einer quer zur optischen Achse (**10**) leicht geneigten Fläche, die von einer Vielzahl radial von der optischen Achse (**10**) wegführenden und auf dem Umfang regelmäßig verteilten Öffnungen (**14**) durchbrochen sind.

Die Breite der Öffnungen (**14**) in Umfangsrichtung sei mit B bezeichnet, der Abstand der Öffnungen (**14**) in Umfangsrichtung mit *W* und die Breite des Materialsteges (**15**) zwischen den Öffnungen (**14**) in Umfangsrichtung sei *S* = *W - B*.

Beim in Elektronenstrahlrichtung letzten Target (**5**) kann auf die Öffnungen (**14**) verzichtet werden.

Die Breite der Öffnungen (**14**) B sollte so gewählt werden, dass *W*/*S* = *W*/*(W - B)* = *N* gilt, wobei *N* die Anzahl der Targets (**5**) bezeichnet (siehe Fig. 2). Die Anordnung der *N* hintereinander liegenden Targets (**5**) erfolgt in Bezug auf die optische Achse (**10**) mit einem winkelmäßigen Versatz von Δα/N relativ zu einem für alle Targets (**5**) identisch festgelegten Bezugswinkel mit dem Bogenabstand Δα zweier benachbarter Öffnungen (**14**).

Überstreicht der Elektronenstrahl (**4**) den Materialsteg (**15**) zwischen den Öffnungen (**14**) des in Elektronenstrahlrichtung vordersten Targets (**5**), so wird in der oberflächlichen Metallschicht durch Elektronenbremsung Röntgenstrahlung innerhalb eines durch die Fokussierung bestimmten Brennflecks (**13**) erzeugt. Überstreicht der Elektronenstrahl (**4**) eine der Öffnungen (**14**) des in Elektronenstrahlrichtung vordersten Targets (**5**), so tritt er durch diese hindurch und fällt auf das darunterliegende zweite Target (**5**). Durch den leichten Winkelversatz dieses Targets (**5**) im Bezug zum ersten Target (**5**) überstreicht der Elektronenstrahl (**4**) wiederum kurzzeitig einen Materialsteg (**15**) zwischen den Öffnungen (**14**) des zweiten Targets (**5**), womit Röntgenstrahlung in der zweiten Durchstrahlungsebene erzeugt wird. Bei weiterer Bewegung des Elektronenstrahls (**4**) tritt dieser durch die Öffnungen (**14**) des ersten und zweiten Targets (**5**) und überstreicht den Materialsteg (**15**) zwischen den Öffnungen (**14**) des in Elektronenstrahlrichtung liegenden dritten Targets (**5**). Dies setzt sich entsprechend der Anzahl vorhandener Targets (**5**) fort, bis der Elektronenstrahl (**4**) wieder einen Materialsteg (**15**) des obersten Targets (**5**) überstreicht.

Varianten der Targetbeaufschlagung durch den Elektronenstrahl (**4**) ergeben sich aus der Art der Strahlfokussierung. Einmal ist es möglich, den Elektronenstrahl (**4**) möglichst scharf zu fokussieren, so dass der Durchmesser des Brennflecks (**13**) kleiner als die Breite des Materialstegs (**15**) S ist. Dies bedeutet, dass Durchstrahlungsprojektionen in den einzelnen Durchstrahtungsebenen zeitlich nacheinander aufgezeichnet werden, da die Bremsstrahlungserzeugung immer nur auf einem Target (**5**) stattfindet. Für eine solche Variante ist es unter Umständen vorteilhaft, den Elektronenstrahl (**4**) während seiner kreisförmigen Bewegung entsprechend des Abstandes zum beaufschlagten Target (**5**) elektromagnetisch durch die Fokussierspulen des elektronenoptischen Systems (**3**) nachzufokussieren.

Eine weitere Variante besteht darin, den Brennfleck (**13**) sehr groß zu gestalten, beispielsweise in Größenordnung des Abstandes der Öffnungen (**14**) *W*. In diesem Fall erzeugt der Elektronenstrahl (**4**) mit einem Teil seines wirksamen Querschnitts zeitgleich Brennflecke (**13**) auf mehreren Targets (**5**). Dies ermöglicht eine gleichzeitige Erfassung von Durchstrahlungsprojektionen in einer Vielzahl von Durchstrahlungsebenen, allerdings auf Kosten einer entsprechend verminderten Röntgenstrahlungsleistung pro Durchstrahlungsebene.

Zur Erfassung der durch das Untersuchungsobjekt durchdringenden Röntgenstrahlung wird koplanar zu jedem Target ein kreisförmiger oder teilkreisförmiger Röntgendetektorbogen (**6**) angeordnet. Dieser besteht aus einer Vielzahl zu einem Bogen aneinandergereihter Einzeldetektoren. Der Röntgendetektorbogen (**6**) ist mit einer im Bild nicht dargestellten Spezialelektronik verbunden, die die Signale der Einzeldetektoren des Röntgendetektorbogens (**6**) sehr schnell erfasst und speichert.

Targets (**5**) und Röntgendetektorbögen (**6**) sind ebenenweise mit einem leichten axialen Versatz zueinander angeordnet. Sind Targets (**5**) oder Röntgendetektorbögen (**6**) nur teilkreisförmig ausgeführt, so sind Bogenlänge und winkelmäßige Anordnung dieser Komponenten so auszuführen, dass eine, dem Fachmann geläufige, vollständige winkelmäßige Abtastung des Radonraums der Durchstrahlungsebene gewährleistet wird.

Dem Fachmann ebenfalls als geläufig vorausgesetzt werden folgende Ausgestaltungsvarianten:
- Durch die leichte Neigung der dem Elektronenstrahl (**4**) zugewandten Targetoberfläche relativ zur Durchstrahlungsebene kann die Projektion des Brennflecks (**13**) in Richtung der optischer Achse (**10**) verkleinert werden.
- Durch Anordnung von Detektorblenden (**9**) vor den Röntgendetektorbögen (**6**) kann der Eintrag von Streustrahlung bzw. der Eintrag von Strahlung, die zeitgleich auf Targets (**5**) außerhalb der betrachteten Durchstrahlungsebene erzeugt wird, unterbunden werden.

### Bezugszeichenliste

- 1: Vakuumkammer
- 2: Elektronenkanone
- 3: Elektronenoptisches System
- 4: Elektronenstrahl
- 5: Target
- 6: Röntgendetektorbogen
- 7: Aufnahmeöffnung für das Untersuchungsobjekt
- 8: Röntgenstrahlaustrittsfenster
- 9: Detektorblenden
- 10: Optische Achse
- 11: Elektronenstrahlkegel
- 12: Ablenkpunkt
- 13: Brennfleck
- 14: Öffnung
- 15: Materialsteg

## Patentansprüche

1. Anordnung zur Elektronenstrahltomographie, bestehend aus
- Mitteln (**2**, **3**) zur Erzeugung, Fokussierung und Ablenkung eines Elektronenstrahls (**4**) innerhalb einer Vakuumkammer (**1**),
- innerhalb der Vakuumkammer (**1**) in Strahlrichtung hintereinander angeordneten ringförmigen oder teilringförmigen Targets (**5**) zur Bremsung des Elektronenstrahls (**4**),
- koplanar und mit leichtem axialem Versatz zu den Targets (**5**) angeordnete Röntgendetektorbögen (**6**), bestehend aus aneinandergereihten Einzeldetektoren,
- einer Aufnahmeöffnung (**7**) für das Untersuchungsobjekt
- Detektorblenden (**9**) zur Unterdrückung von Streustrahlung,
- zu jedem Target (**5**) ein Röntgendetektorbogen (**6**) koplanar und in radialerRichtung vor oder hinter dem jeweiligen Target (**5**) angeordnet ist
, **dadurch gekennzeichnet, daß**
- jedes Target (**5**) von Öffnungen (**14**) gegebener Breite und regelmäßiger Anordnung in Umfangsrichtung durchbrochen ist,
- die Öffnungen (**14**) in den Targets (**5**) jeweils auf einer Bahnkurve liegen, die den Schnitt der Mantelfläche des Elektronenstrahlkegels (**11**) mit dem jeweiligen Target (**5**) bildet, wobei die Spitze des Elektronenstrahlkegels (**11**) durch den Ablenkpunkt (**12**) des Elektronenstrahls (**4**) und dessen Mantelfläche durch die Bahnen Elektronenstrahls (**4**) im Raum definiert sind,
- die in Strahlrichtung hinter dem vordersten Target (**5**) folgenden Targets (**5**) mit jeweils einem kleinen Winkelversatz zum jeweils davorliegenden Target (**5**) bezüglich der optischen Achse (**10**) angeordnet sind, so dass ein entlang der Mantelfläche des Elektronenstrahlkegels (**11**) zirkulierender Elektronenstrahl (**4**) mit mindestens einem Teil seines Querschnitts nacheinander die Materialstege (**15**) zwischen den Öffnungen (**14**) aller Targets (**5**) bestrahlt.

2. Anordnung nach Anspruch 1, **dadurch gekennzeichnet, dass** der Elektronenstrahldurchmesser kleiner als die Breite des Materialstegs (**15**) ist, so dass zu jedem Zeitpunkt jeweils nur auf einem der Target (**5**) ein Brennfleck (**13**) mit der vollen Bremsstrahlungsleistung entsteht.

3. Anordnung nach Anspruch 1, **dadurch gekennzeichnet, dass** der Elektronenstrahldurchmesser größer als die Breite des Materialstegs (**15**) ist, so dass zu jedem Zeitpunkt auf mehreren Targets (**5**) ein Brennfleck (**13**) mit einem Teil der Bremsstrahlungsleistung entsteht.

## Claims

1. An assembly for electron beam tomography, comprising:
- a device (2, 3) for generating, focusing, and deflecting an electron beam (4) within a vacuum chamber (1);
- annular or partly annular targets (5) arranged successively along a beam direction within said vacuum chamber (1) for decelerating the electron beam (4);
- a plurality of X-ray detector arcs (6) formed of individual detectors placed next to one another, said X-ray detector arcs being disposed in a coplanar relation and with a slight axial offset to said targets (5);
- a recording aperture (7) for an examination object; and
- detector diaphragms (9) configured to suppress scattered radiation;
- an X-ray detector arc (6) for each said target (5) disposed in coplanar fashion and in a radial direction in front of or behind the respective said target (5),
**characterized in that**,
- each target (5) having openings (14) of a given width formed therein and material webs (15) there between, regularly arranged in a circumferential direction;
- said openings (14) in said targets (5) being respectively situated on a path formed by a cross section of a shell of an electron beam cone (11) with the respective said target (5), with a tip of the electron beam cone (11) being defined in space by a deflection point (12) of the electron beam (4) and a shell thereof through the paths of the electron beam (4);
- said targets (5), following a forward-most target (5) in the beam direction, respectively being disposed with a small angular offset with respect to an optical axis (10) to the respective said target (5) situated in front, and with an electron beam (4) circulating along the shell of the electron beam cone (11) successively irradiating said material webs (15) between said openings (14) of all targets (5) with at least part of cross section thereof.

2. The assembly according to claim 1, wherein a diameter of the electron beam is less than a width of said material web (15) such that, at any one time, a focal point (13) with a full *Bremsstrahlung* power is generated in each case on only one of said targets (5).

3. The assembly according to claim 1, wherein the electron beam diameter is greater than a width of said material web (15) such that, at any one time, a focal point (13) with part of a Bremsstrahlung power is generated on a plurality of targets (5).

## Revendications

1. Arrangement de tomographie à faisceau d'électrons composé de
- des moyens (2, 3) pour générer, concentrer et dévier un faisceau d'électrons (4) à l'intérieur d'une chambre sous vide (1),
- des cibles (5) de forme annulaire ou partiellement annulaire disposées les unes derrières les autres dans le sens du faisceau à l'intérieur de la chambre sous vide (1), pour freiner le faisceau d'électrons (4),
- des arcs détecteurs de rayons X (6) disposés dans le même plan et avec un léger décalage axial par rapport aux cibles (5), composés de détecteurs individuels alignés les uns contre les autres,
- une ouverture d'accueil (7) pour l'objet examiné,
- des obturateurs de détecteur (9) pour inhiber le rayonnement diffusé,
- pour chaque cible (5), un arc détecteur de rayons X (6) disposé dans le même plan et devant ou derrière la cible (5) respective dans le sens radial,
**caractérisé en ce que**
- chaque cible (5) est traversée par des ouvertures (14) de largeur donnée et disposées régulièrement dans le sens du pourtour,
- les ouvertures (14) dans les cibles (5) se trouvent respectivement sur une orbite qui forme l'intersection de la surface d'enveloppe du cône du faisceau d'électrons (11) avec la cible (5) respective, la pointe du cône du faisceau d'électrons (11) étant définie par le point de déviation (12) du faisceau d'électrons (4) et sa surface d'enveloppe par les trajectoires du faisceau d'électrons (4) dans l'espace,
- les cibles (5) qui suivent la cible (5) la plus à l'avant dans le sens du faisceau sont respectivement disposées avec un décalage angulaire petit par rapport à la cible (5) qui se trouve à chaque fois à l'avant en référence à l'axe optique (10), de sorte qu'un faisceau d'électrons (4) qui circule le long de la surface d'enveloppe du cône du faisceau d'électrons (11) irradie l'un après l'autre les barreaux de matière (15) entre les ouvertures (14) de toutes les cibles (5) avec au moins une partie de sa section transversale.

2. Arrangement selon la revendication 1, **caractérisé en ce que** le diamètre du faisceau d'électrons est inférieur à la largeur du barreau de matière (15), de sorte qu'à chaque instant il ne se produit à chaque fois une tache cathodique (13) avec la pleine puissance du rayonnement de freinage que sur l'une des cibles (5).

3. Arrangement selon la revendication 1, **caractérisé en ce que** le diamètre du faisceau d'électrons est supérieur à la largeur du barreau de matière (15), de sorte qu'à chaque instant il se produit une tache cathodique (13) avec une partie de la puissance du rayonnement de freinage sur plusieurs cibles (5).
